(19) Europäisches Patentamt
European Patent Office
Office européen des brevets

(11) EP 0 895 779 B1

(12) **FASCICULE DE BREVET EUROPEEN**

(45) Date de publication et mention de la délivrance du brevet:
**20.08.2003 Bulletin 2003/34**

(51) Int Cl.$^7$: **A61K 7/48**, A61K 7/135

(21) Numéro de dépôt: **98401544.6**

(22) Date de dépôt: **23.06.1998**

(54) **Utilisation de monoesters d'arbutine comme agent dépigmentant**

Verwendung von Arbutin Monoestern als Hautdepigmentierungsmittel

Use of monesters of arbutin as skin depigmentation agent

(84) Etats contractants désignés:
**DE ES FR GB IT**

(30) Priorité: **08.07.1997 FR 9708674**

(43) Date de publication de la demande:
**10.02.1999 Bulletin 1999/06**

(73) Titulaire: **L'OREAL**
**92583 Clichy Cédex (FR)**

(72) Inventeur: **Philippe, Michel**
**91320 Wissous (FR)**

(74) Mandataire: **Tezier Herman, Béatrice**
**L'OREAL-DPI**
**6 rue Bertrand Sincholle**
**92585 Clichy Cédex (FR)**

(56) Documents cités:
**EP-A- 0 597 776**
**FR-A- 2 577 805**
**US-A- 4 526 779**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 895 779 B1

## Description

La présente invention se rapporte à l'utilisation de monoesters d'arbutine comme agent dépigmentant ou blanchissant dans une composition à application topique, notamment cosmétique et/ou dermatologique, ainsi qu'à l'utilisation de ces esters d'arbutine dans et/ou pour la fabrication d'une composition cosmétique et/ou dermatologique, comme inhibiteur de la tyrosinase et/ou de la synthèse de la mélanine. La présente invention se rapporte aussi à un procédé de dépigmentation et/ou de blanchiment de la peau, des poils et/ou des cheveux, à l'aide de tels monoesters d'arbutine.

La couleur de la peau est fonction de différents facteurs et notamment des saisons de l'année, de la race et du sexe, et elle est principalement déterminée par la concentration de mélanine produite par les mélanocytes. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

De la même manière, la couleur des poils et des cheveux est due à la mélanine. Lorsque les poils ou les cheveux sont foncés, certaines personnes désirent voir ceux-ci plus claires. Ceci est particulièrement intéressant pour les poils qui sont moins visibles lorsqu'ils sont clairs que lorsqu'ils sont foncés.

Depuis plusieurs années, on cherche à diminuer et/ou ralentir la production de mélanine en vue de dépigmenter ou blanchir la peau en agissant sur un ou plusieurs des points de la synthèse biochimique intracellulaire de la mélanine.

Le mécanisme de formation de la pigmentation de la peau, des poils et des cheveux, c'est-à-dire de la formation de la mélanine est particulièrement complexe et fait intervenir schématiquement les principales étapes suivantes

Tyrosine ---> Dopa ---> Dopaquinone ---> Dopachrome ---> Mélanine

La tyrosinase (monophénol dihydroxyl phénylalanine : oxygen oxydo-reductase EC 1,14,18,1) est l'enzyme essentielle intervenant dans cette suite de réactions. Elle catalyse notamment la réaction de transformation de la tyrosine en Dopa (dihydroxyphénylalanine) et la réaction de transformation de la Dopa en dopaquinone. Cette tyrosinase n'agit que lorsqu'elle est à l'état de maturation sous l'action de certains facteurs biologiques.

Une substance est reconnue comme dépigmentante si elle agit directement sur la viabilité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut ainsi être bloquée et assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

Pour pallier aux inconvénients mentionnés ci-dessus, il a été envisagé par exemple d'utiliser des dérivés des composés actifs. Ainsi, le document US-A-4,526,779 décrit l'utilisation d'esters d'acide gras et d'hydroquinone comme agents dépigmentants. Malheureusement, ces dérivés sont moins actifs que l'hydroquinone.

On a ainsi cherché des substances qui n'interviennent pas dans le mécanisme de la mélanogénèse, mais qui agissent en amont sur la tyrosinase en empêchant son activation et sont, de ce fait, beaucoup moins toxiques. On utilise couramment comme inhibiteur de l'activation de la tyrosinase, l'acide kojique qui complexe le cuivre présent dans le site actif de cette enzyme. Malheureusement, ce composé peut provoquer des réactions d'allergie ("Contact allergy to kojic acid in skin care products", Nakagawa M. et al., in Contact Dermatitis, Jan. 95, Vol 42 (1), pp.9-13). En outre, ce composé est instable en solution, ce qui complique quelque peu la fabrication de la composition le contenant.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines

leucodermies, telles que le vitiligo. Pour ces dernières où les cicatrisations peuvent aboutir à une cicatrice donnant à la peau un aspect plus blanc et les leucodermies, à défaut de pouvoir repigmenter la peau lésée, on achève de dépigmenter les zones de peau normale résiduelle pour donner à l'ensemble de la peau une teinte blanche homogène.

Aussi, il subsiste le besoin d'un nouvel agent blanchissant de la peau humaine, des poils et/ou des cheveux à action aussi efficace que ceux connus, mais n'ayant pas leurs inconvénients, c'est-à-dire qui soit non irritant, non toxique et/ou non allergisant pour la peau et qui soit stable dans une composition.

La demanderesse a trouvé de manière inattendue que certains esters d'arbutine présentaient la propriété d'inhiber la synthèse de la mélanine et étaient susceptibles d'agir ainsi sur la pigmentation et les taches de la peau sans toxicité. Ceci est d'autant plus surprenant que la fonction hydroxyle aromatique libre de l'arbutine, importante dans la reconnaissance enzymatique, est, dans ces composés, masquée par un résidu ester, et que l'activité dépigmentante est néanmoins conservée, alors que ce même hydroxyle aromatique bloqué par un résidu sucré tel que le glucose ne permet plus à la molécule de garder son activité dépigmentante. Ainsi, par exemple, le di-O-β-glucopyranose-1,4-hydroquinone ne présente aucune activité dépigmentante.

Certes, le document EP-A-597776 décrit les composés utilisés selon l'invention et leurs applications dans les domaines cosmétique, pharmaceutique, bucco-dentaire ou alimentaire. Toutefois, il ne décrit pas leur activité dépigmentante.

L'invention a donc pour objet l'utilisation d'au moins un monoester d'arbutine de formule (I) :

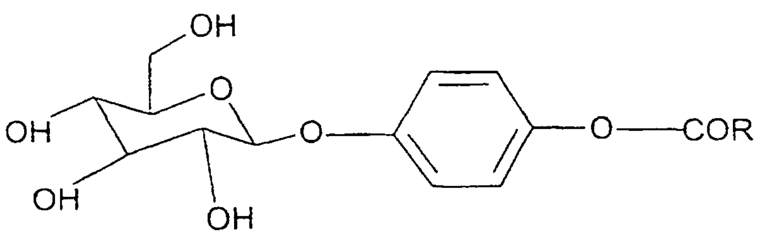

(I)

dans laquelle :

R représente un radical alkyle saturé, linéaire ou ramifié, comportant de 5 à 21 atomes de carbone, un radical alcényle comportant de 5 à 21 atomes de carbone ou un radical alcapolyényle comportant de 9 à 21 atomes de carbone, dans et/ou pour la fabrication d'une composition cosmétique et/ou dermatologique pour dépigmenter et/ou blanchir la peau humaine et/ou enlever les taches pigmentaires de la peau et/ou dépigmenter les poils et/ou les cheveux.

Enfin, l'invention a pour objet un procédé cosmétique et/ou dermatologique de dépigmentation et/ou blanchiment de la peau humaine, des poils ou des cheveux consistant à appliquer sur la peau, les poils ou les cheveux, un ester de formule (I) dans un milieu physiologiquement acceptable.

Selon la présente invention, le radical alkyle linéaire ou ramifié comporte de préférence de 6 à 18 atomes de carbone. De préférence, le radical RCO où R est un radical alkyle, représente un radical choisi parmi les radicaux hexanoyle, décanoyle, dodécanoyle et hexadécanoyle.

On entend par radical alcényle (ou alcoylène), un radical insaturé présentant une insaturation éthylénique. Le radical alcényle comporte de préférence de 6 à 18 atomes de carbone. De préférence, le radical RCO où R est un radical alcényle représente le radical oléoyle.

On entend par radical alcapolyényle un radical insaturé présentant plusieurs insaturations éthyléniques, notamment présentant deux ou trois insaturations éthyléniques. Le radical alcapolyényle est en particulier un radical alcadiényle ou alcatriényle et comporte de préférence de 12 à 20 atomes de carbone. Le radical RCO où R est un radical alcapolyényle est plus particulièrement le radical linoléoyle.

Parmi les monoesters d'arbutine correspondant à la formule générale (I), on peut notamment citer le 4-hexanoyl-oxy-phényl-β-D-glucose, le 4-décanoyl-oxy-phényl-β-D-glucose, le 4-dodécanoyl-oxy-phényl-β-D-glucose, le 4-hexadécanoyl-oxy-phényl-β-D-glucose, le 4-oléoyl-oxy-phényl-β-D-glucose et le 4-linoléoyl-oxy-phényl-β-D-glucose. On peut aussi utiliser un mélange de ces composés.

Dans les compositions dépigmentantes selon l'invention, les moesters de formule (I) doivent être en une quantité efficace pour assurer le résultat escompté. Cette quantité peut aller par exemple de 0,001 à 10 % et de préférence de 0,005 à 5 % du poids total de la composition.

La composition contenant les composés selon l'invention comporte un milieu physiologiquement acceptable

approprié pour une application topique, c'est-à-dire compatible avec la peau, le cuir chevelu et les cheveux, et constitue une composition à application topique, cosmétique et/ou dermatologique, blanchissante et/ou dépigmentante.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'un produit à deux phases, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick.

La composition selon l'invention peut comprendre tous les ingrédients classiquement utilisés dans le domaine cosmétique ou dermatologique, dans des concentrations habituelles. Ces ingrédients sont en particulier choisis parmi les corps gras, les conservateurs, les gélifiants, les parfums, les émulsionnants, l'eau, les antioxydants, les charges, les actifs (hydrophiles ou lipophiles), les filtres et leurs mélanges.

Comme corps gras utilisables dans l'invention, on peut citer les huiles minérales (huile de vaseline), les huiles d'origine végétale, les huiles d'origine animale, les huiles de synthèse (myristate d'isopropyle, ethyl-2 hexanoate de cétylstéaryle), les huiles de silicone et les huiles fluorées. On peut aussi utiliser des alcools gras (alcool hexyl-2.décylique-1, alcool cétylique), des acides gras (acide stéarique), des cires, et leurs mélanges.

Comme émulsionnants utilisables dans l'invention, on peut citer par exemple les esters d'acide gras et de polyéthylène glycol tels que les stéarates de polyéthylèneglycol, et les esters d'acide gras et de glycéryle tels que le stéarate de glycéryle, et leurs mélanges. On peut citer par exemple le mélange de stéarate de PEG-100 et de stéarate de glycéryle, vendu sous le nom d'Arlacel 165 par la société ICI.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les hydratants tels que les polyols (glycérine, propylène glycol), les vitamines, les agents kératolytiques et/ou desquamants (acide salicylique et ses dérivés, alpha-hydroxyacides, acide ascorbique et ses dérivés), les agents anti-inflammatoires, les agents apaisants et leurs mélanges. En cas d'incompatibilité, ces actifs peuvent être incorporés dans des sphérules, notamment des vésicules ioniques ou non-ioniques et/ou des nanoparticules (nanocapsules et/ou nanosphères), de manière à les isoler les uns des autres dans la composition.

Un test a mis en évidence l'activité de composés utilisés selon l'invention comme dépigmentant, comparativement à l'activité de l'acide kojique.

Le test est réalisé sur coculture de kératinocytes et de mélanocytes selon le protocole décrit dans la demande de brevet FR-A-2734825 déposée par la demanderesse et dans l'article de R. Schmidt, P. Krim et M. Requin, Analyses Biochimiques 235(2), 113-18, (1996).

Pour chaque composé testé, il est déterminé la valeur de IC50, c'est-à-dire la concentration micromolaire (μM) pour laquelle est observée 50 % d'inhibition de la mélanogénèse .

| Composé testés | IC50 |
|---|---|
| 4-dodécanoyl-oxy-phényl-β-D-glucose | 10 μM |
| 4-oléoyl-oxy-phényl-β-D-glucose | 50 μM |
| 4-hexadécanoyl-oxy-phényl-β-D-glucose | 200 μM |
| Acide kojique | 500 μM |

L'arbutine présente dans ce test une IC 50 équivalente à celle de l'acide kojique.

Ce tableau montre que les composés de formule (I) utilisés selon l'invention ont une plus grande efficacité que l'acide kojique. En outre, ils ont l'avantage de ne pas présenter de cytotoxicité à l'égard des kératinocytes et les mélanocytes, défaut majeur des dépigmentants existants.

Par ailleurs, ce test a montré que le di-O-β-glucopyranose-1,4-hydroquinone ne présente aucune activité dépigmentante.

L'invention va maintenant être illustrée à l'aide de l'exemple qui suit. Les concentration sont données en pourcentage en poids.

**Exemple 1: Emulsion huile-dans-eau**

- 4-hexadécanoyl-oxy-phényl-β-D-glucose 0,05 %
- Ethyl-2 hexanoate de cétylstearyle/myristate d'isopropyle (90/10) 2 %
- huile de vaseline 7,5 %
- conservateur 0,2 %
- polymère carboxyvinylique 0,35 %
- triéthanolamine 1,05 %
- glycérine 3 %
- propylène glycol 8 %
- alcool hexyl-2 décylique-1 1 %
- alcool cétylique 0,1 %
- acide stéarique 1,4 %
- Arlacel 165 2 %
- eau qsp 100 %

La crème obtenue utilisée en application quotidienne, permet d'obtenir un blanchiment de la peau.

**Revendications**

**1.** Utilisation cosmétique d'au moins un monoester d'arbutine de formule (I):

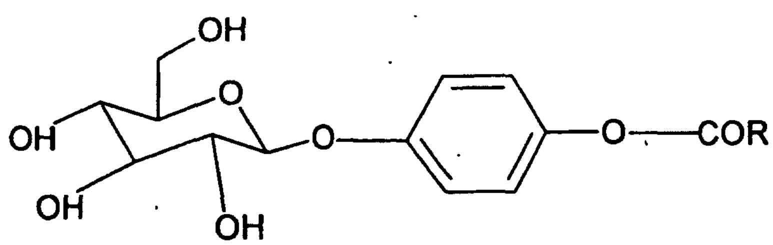

(I)

dans laquelle :

R représente un radical alkyle saturé, linéaire ou ramifié, comportant de 5 à 21 atomes de carbone, un radical alcényle comportant de 5 à 21 atomes de carbone ou un radical alcapolyényle comportant de 9 à 21 atomes de carbone, pour dépigmenter et/ou blanchir la peau humaine et/ou enlever les taches pigmentaires de la peau et/ou dépigmenter les poils et/ou les cheveux.

**2.** Utilisation d'au moins un monoester d'arbutine de formule (I) :

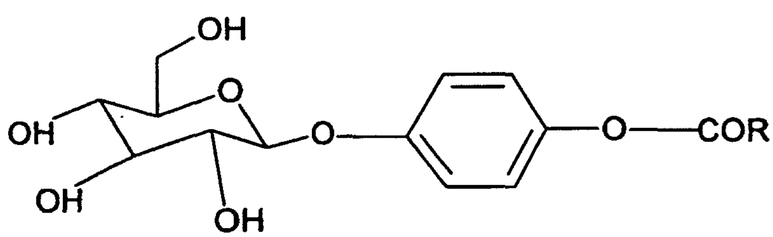

(I)

dans laquelle

R représente un radical alkyle saturé, linéaire ou ramifié, comportant de 5 à 21 atomes de carbone, un radical

alcényle comportant de 5 à 21 atomes de carbone ou un radical alcapolyényle comportant de 9 à 21 atomes de carbone, en tant qu'agent dépigmentant, pour la fabrication d'une composition dermatologique destinée à dépigmenter et/ou blanchir la peau humaine et/ou enlever les taches pigmentaires de la peau et/ou dépigmenter les poils et/ou les cheveux.

**3.** Utilisation selon la revendication 1 ou 2, **caractérisée en ce que** R représente un radical alkyle ou alcényle comportant de 6 à 18 atomes de carbone.

**4.** Utilisation selon l'une quelconque des revendications précédentes, **caractérisée en ce que** RCO représente un radical choisi parmi les radicaux hexanoyle, décanoyle, dodécanoyle, hexadécanoyle, oléoyle et linoléoyle.

**5.** Utilisation selon l'une quelconque des revendications précédentes, **caractérisée en ce que** le monoester de formule (I) est choisi parmi le 4-hexanoyl-oxy-phényl-β-D-glucose, le 4-décanoyl-oxy-phényl-β-D-glucose, le 4-dodécanoyl-oxy-phényl-β-D-glucose, le 4-hexadécanoyl-oxy-phényl-β-D-glucose, le 4-oléoyl-oxy-phényl-β-D-glucose, le 4-linoléoyl-oxy-phényl-β-D-glucose et leurs mélanges.

**6.** Utilisation selon l'une quelconque des revendications précédentes, **caractérisée en ce que** le monoester de formule (I) est présent en une quantité allant de 0,001 à 10 % du poids total de la composition.

**7.** Utilisation selon l'une quelconque des revendications précédentes, **caractérisée en ce que** la composition comprend, en outre, au moins un ingrédient choisi parmi les corps gras, les conservateurs, les gélifiants, les parfums, les émulsionnants, l'eau, les antioxydants, les charges, les filtres, les actifs et leurs mélanges.

**8.** Utilisation selon la revendication précédente, **caractérisée en ce que** l'actif est choisi parmi les hydratants, les vitamines, les agents kératolytiques et/ou desquamants, les agents anti-inflammatoires, les agents apaisants et leurs mélanges.

## Patentansprüche

**1.** Kosmetische Verwendung mindestens eines Arbutinmonoesters der Formel (I):

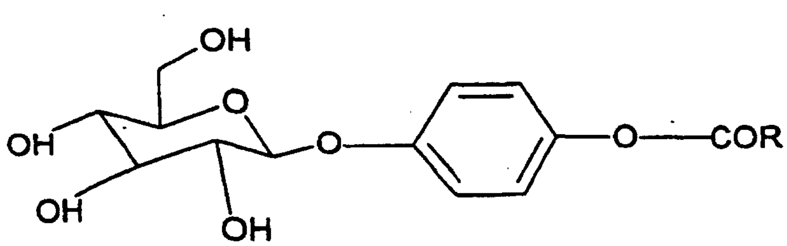

(I),

worin R eine gesättigte, geradkettige oder verzweigte Alkylgruppe mit 5 bis 21 Kohlenstoffatomen, eine Alkenylgruppe mit 5 bis 21 Kohlenstoffatomen oder eine Alkapolyenylgruppe mit 9 bis 21 Kohlenstoffatomen bedeutet, um die menschliche Haut zu depigmentieren und/oder zu bleichen und/oder Pigmentflecken der Haut zu entfernen und/oder die Körperhaare und/oder die Haare zu depigmentieren.

**2.** Verwendung mindestens eines Arbutinmonoesters der Formel (I):

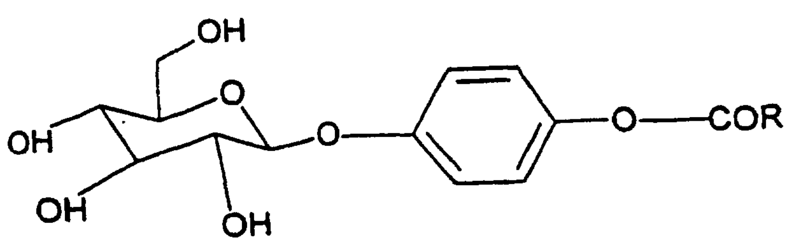

(I),

worin R eine gesättigte, geradkettige oder verzweigte Alkylgruppe mit 5 bis 21 Kohlenstoffatomen, eine Alkenylgruppe mit 5 bis 21 Kohlenstoffatomen oder eine Alkapolyenylgruppe mit 9 bis 21 Kohlenstoffatomen bedeutet, als Depigmentierungsmittel für die Herstellung einer dermatologischen Zusammensetzung, die dazu vorgesehen ist, die menschliche Haut zu depigmentieren und/oder zu bleichen und/oder Pigmentflecken der Haut zu entfernen und/oder die Körperhaare und/oder die Haare zu depigmentieren.

**3.** Verwendung nach Anspruch 1 oder 2, **dadurch gekennzeichnet, dass** R eine Alkyl- oder Alkenylgruppe mit 6 bis 18 Kohlenstoffatomen ist.

**4.** Verwendung nach einem der vorhergehenden Ansprüche, **dadurch gekennzeichnet, dass** RCO eine Gruppe bedeutet, die unter den Gruppen Hexanoyl, Decanoyl, Dodecanoyl, Hexadecanoyl, Oleoyl und Linoleoyl ausgewählt ist.

**5.** Verwendung nach einem der vorhergehenden Ansprüche, **dadurch gekennzeichnet, dass** der Monoester der Formel (I) unter 4-Hexanoyl-oxy-phenyl-β-D-glucose, 4-Decanoyl-oxy-phenyl-β-Dglucose, 4-Dodecanoyl-oxy-phenyl-β-D-glucose, 4-Hexadecanoyloxy-phenyl-β-D-glucose, 4-Oleoyl-oxy-phenyl-β-D-glucose, 4-Linoleoyl-oxy-phenyl-β-D-glucose und deren Gemischen ausgewählt ist.

**6.** Verwendung nach einem der vorhergehenden Ansprüche, **dadurch gekennzeichnet, dass** der Monoester der Formel (I) in einer Menge von 0,001 bis 10 % des Gesamtgewicht der Zusammensetzung vorliegt.

**7.** Verwendung nach einem der vorhergehenden Ansprüche, **dadurch gekennzeichnet, dass** die Zusammensetzung ferner mindestens einen Bestandteil enthält, der unter den Fettsubstanzen, Konservierungsmitteln, Gelbildnern, Parfums, Emulgatoren, Wasser, Antioxidantien, Füllstoffen, Filtern, Wirkstoffen und deren Gemischen ausgewählt ist.

**8.** Verwendung nach dem vorhergehenden Anspruch, **dadurch gekennzeichnet, dass** der Wirkstoff unter den Hydratisierungsmitteln, Vitaminen, keratolytischen und/oder abschuppenden Wirkstoffen, entzündungshemmenden Wirkstoffen, reizlindernden Wirkstoffen und deren Gemischen ausgewählt ist.

## Claims

**1.** Cosmetic use of at least one arbutin monoester of formula (I):

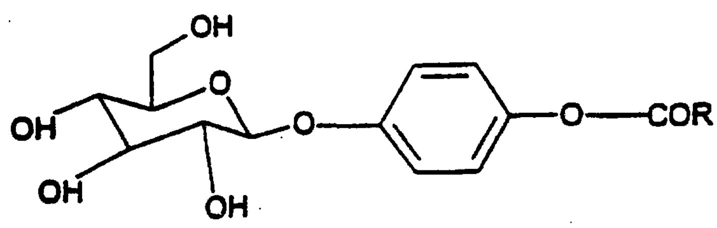

(I)

in which:

R represents a linear or branched, saturated alkyl radical containing from 5 to 21 carbon atoms, an alkenyl radical containing from 5 to 21 carbon atoms or an alcapolyenyl radical containing from 9 to 21 carbon atoms, for depigmenting and/or bleaching human skin and/or for removing pigmentation marks from the skin and/or for depigmenting hairs and/or head hair.

**2.** Use of at least one arbutin monoester of formula (I):

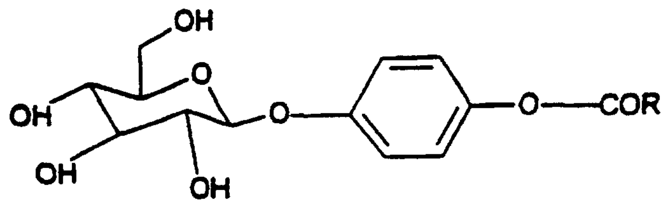

(I)

in which:

R represents a linear or branched, saturated alkyl radical containing from 5 to 21 carbon atoms, an alkenyl radical containing from 5 to 21 carbon atoms or an alcapolyenyl radical containing from 9 to 21 carbon atoms, as depigmenting agent, for the manufacture of a dermatological composition intended for depigmenting and/or bleaching human skin and/or for removing pigmentation marks from the skin and/or for depigmenting hairs and/or head hair.

**3.** Use according to Claim 1 or 2, **characterized in that** R represents an alkyl or alkenyl radical containing from 6 to 18 carbon atoms.

**4.** Use according to any one of the preceding claims, **characterized in that** RCO represents a radical chosen from hexanoyl, decanoyl, dodecanoyl, hexadecanoyl, oleoyl and linoleoyl radicals.

**5.** Use according to any one of the preceding claims, **characterized in that** the monoester of formula (I) is chosen from 4-hexanoyloxyphenyl-β-D-glucose, 4-decanoyloxyphenyl-β-D-glucose, 4-dodecanoyloxyphenyl-β-D-glucose, 4-hexadecanoyloxyphenyl-β-D-glucose, 4-oleoyloxyphenyl-β-D-glucose and 4-linoleoyloxyphenyl-β-D-glucose and mixtures thereof.

**6.** Use according to any one of the preceding claims, **characterized in that** the monoester of formula (I) is present in an amount ranging from 0.001 to 10% of the total weight of the composition.

**7.** Use according to any one of the preceding claims, **characterized in that** the composition also comprises at least one ingredient chosen from fatty substances, preserving agents, gelling agents, fragrances, emulsifiers, water, antioxidants, fillers, screening agents and active agents, and mixtures thereof.

**8.** Use according to the preceding claim, **characterized in that** the active agent is chosen from moisturizers, vitamins, keratolytic agents and/or desquamating agents, anti-inflammatory agents and calmants, and mixtures thereof.